Europäisches Patentamt

European Patent Office

Office européen des brevets

(19)

(11) Numéro de publication : **0 335 785 B1**

(12) # FASCICULE DE BREVET EUROPEEN

(45) Date de publication du fascicule du brevet :
**22.01.92 Bulletin 92/04**

(51) Int. Cl.⁵ : **C08F 246/00,** A61K 9/22, G02B 1/04

(21) Numéro de dépôt : **89400837.4**

(22) Date de dépôt : **24.03.89**

(54) **Polymères supports de produits actifs relargables, de type hydrogel, compositions correspondantes et leur procédé de préparation.**

(30) Priorité : **28.03.88 FR 8804041**

(43) Date de publication de la demande :
**04.10.89 Bulletin 89/40**

(45) Mention de la délivrance du brevet :
**22.01.92 Bulletin 92/04**

(84) Etats contractants désignés :
**CH DE ES GB IT LI**

(56) Documents cités :
**FR-A- 2 190 454**

(73) Titulaire : **ESSILOR INTERNATIONAL, Cie Générale d'Optique**
**1 Rue Thomas Edison, Echat 902**
**F-94028 Créteil Cédex (FR)**

(72) Inventeur : **Bunel, Claude**
**6 Rue Lemaignan**
**F-76420 Bihorel (FR)**
Inventeur : **Meslard, Jean-Claude**
**45 Rue du Maréchal Leclerc**
**F-94410 Saint Maurice (FR)**
Inventeur : **Vairon, Jean-Pierre**
**7 Rue Alfred Nomblot**
**F-92340 Bourg la Reine (FR)**

(74) Mandataire : **Thibon-Littaye, Annick**
**Cabinet A. THIBON-LITTAYE 11 rue de l'Etang**
**F-78160 Marly-le-Roi (FR)**

EP 0 335 785 B1

Jouve, 18, rue Saint-Denis, 75001 PARIS

**Description**

L'invention concerne la préparation de polymères supportant des produits actifs relargables, ces produits actifs étant plus spécialement des principes chimiques actifs tels que les médicaments et les principes actifs analogues. Elle a pour objet une composition d'un tel polymère, constituée d'un copolymère dans lequel le produit actif est intégré et retenu par une liaison chimique. Elle a également pour objet un procédé de préparation d'une telle composition ainsi que les matériaux polymères obtenus par mise en forme et polymérisation d'une telle composition.

Parmi les matériaux supports pouvant servir au relargage de produits actifs tels que des médicaments, l'invention s'intéresse plus particulièrement à ceux où le produit actif est immobilisé provisoirement dans un polymère d'où il est libéré progressivement à l'emploi, sans destruction du matériau de base, de sorte que ce dernier conserve l'essentiel de ses propriétés initiales, hormis bien entendu celles qui sont directement liées au produit actif lorsque celui-ci est épuisé après libération complète.

Le besoin de tels matériaux s'est fait sentir notamment dans le domaine des polymères hydrophiles tels que ceux qui sont utilisés pour la constitution de lentilles de contact. Un relargage du produit actif, tel qu'un principe actif de médicament, qui intervient sans destruction du polymère support de base permet de préserver les qualités optiques des lentilles et leur biocompatibilité, alors que le principe du relargage d'un médicament incorporé dans le polymère permet de rendre celui-ci disponible progressivement, directement dans le milieu où il doit exercer ses effets.

Dans ce domaine, une première voie consistait à emprisonner le médicament physiquement au sein du polymère, sans liaison chimique spéciale, et à provoquer son relargage par simple diffusion à travers le polymère hydrophile en milieu oculaire.

Il est cependant préférable d'intégrer le médicament dans la composition du polymère par copolymérisation de manière à introduire des liaisons chimiques entre la chaîne du polymère et les molécules du produit actif. Il convient par contre alors, que ces liaisons puissent s'ouvrir facilement pour libérer le produit actif, notamment par hydrolyse dans le milieu oculaire, et ce, sans détruire aucunement le polymère dans ses autres propriétés.

Dans ce sens, le brevet français FR-A-2548673 déposé au nom de la demanderesse, a proposé des polymères hydrophiles dans lesquels les molécules d'un composé actif sont fixées sur des fragments latéraux d'une chaîne polymère de base par des liaisons acétal. Cette solution avait l'avantage d'être applicable par exemple au chloramphénicol, médicament particulièrement utile en ophtalmologie. Elle a pour contre l'inconvénient d'exiger que le médicament, dans la molécule d'origine, soit porteur de deux fonctions alcool capables de réagir avec les constituants de la composition polymérique pour former la liaison acétal.

La présente invention vise à éviter cet inconvénient en apportant une solution répondant au mieux aux diverses exigences de la pratique telles qu'elles ont été rappelées ci-dessus. Elle convient spécialement aux applications impliquant un produit actif dont la molécule comporte une fonction acide.

Ainsi, la composition de polymère suppport de produit actif relargable selon l'invention se caractérise en ce qu'elle comporte des constituants classiques d'un hydrogel et un monomère porteur de produit actif copolymérisable avec lesdits constituants, ledit monomère comportant un radical aryle dont une fonction phénol est estérifiée par un composé à fonction acide constituant ledit produit actif.

Dans les applications pratiques de l'invention, les constituants d'un hydrogel sont de préférence ceux d'un hydrogel du type convenant à la fabrication de lentilles de contact, comme le méthacrylate de méthyle, le méthacrylate d'hydroxyéthyle (HEMA), le méthacrylate de glycéryle, la N-vinylpyrrolidone, l'acide acrylique et l'acide méthacrylique, ces différents composés étant utilisés soit seuls, soit plus souvent en mélange, et sous forme de monomères, d'homopolymères ou de copolymères. Par ailleurs, le composé constituant le produit actif est de préférence du type de l'indométhacine, médicament connu dont la formule comporte une fonction acide terminale d'an substituant aliphatique sur un hétérocycle azoté, plus spécialement caractérisé par un cycle benzopyrroline substitué par un radical hydroxyoxyalkyle. Dans ses applications en ophtalmologie, ce médicament a en outre la particularité d'être actif à faible dose. Il est de préférence incorporé dans la composition selon l'invention dans une proportion comprise entre 1 et 10% en poids, notamment entre 2 et 6% en poids, par rapport au poids total de la composition. D'autres composés du même type que l'indométhacine sont le fentiazac et le suprofène.

A partir d'une telle composition, un matériau convenant à la fabrication de lentilles de contact peut être obtenu, par durcissement de la composition dans les conditions classiques de mise en forme et de polymérisation des hydrogels, adaptées en fonction des constituants d'hydrogel et des monomères porteurs de produit actif utilisés dans chaque cas particulier. Le plus souvent, on procède par polymérisation en masse, dans des moules de forme appropriée, après avoir ajouté à la composition un réticulant et un amorceur de polymérisation, dont le choix en nature et en quantité répond à des critères bien connus de l'homme de l'art ; bien évidemment le réticulant et l'amorceur peuvent chacun être en pratique un mélange de plusieurs composés chimiques.

Du point de vue chimique, un tel matériau comporte une chaîne polymérique d'hydrogel contenant des motifs où le produit actif est lié chimiquement à la chaîne polymérique par l'intermédiaire du radical aryle du monomère porteur auquel il est fixé par une liaison ester. On a constaté que cette liaison ester, en combinaison avec la présence du radical aryle qui espace la molécule de produit actif de la chaîne polymérique de base, favorisait considérablement l'hydrolyse de la liaison ester et la libération du produit actif dans les conditions rencontrées habituellement dans le milieu oculaire et pour les faibles doses de produit actif désirées.

Pour permettre sa copolymérisation avec les constituants classiques de l'hydrogel, le monomère porteur de produit actif comporte un groupe polymérisable, qui peut être un groupe insaturé de tout type connu, les plus fréquents étant les groupes vinylique, allylique, acrylique et méthacrylique. Dans le cadre de la présente invention, on accorde cependant une préférence aux monomères constitués par des esters ou amides acryliques ou méthacryliques.

De tels monomères répondent avantageusement à l'une ou l'autre des formules suivantes :

$H_2C=C (R1)-CO-O-R2$

ou $H_2C=C (R1)-CO-N (R3)-R2$

où R1 et R3 sont H ou $CH_3$

et R2 est un radical aryle porteur de la fonction phénol estérifiée par le produit actif, et plus particulièrement un radical hydroxyphényle ainsi estérifié.

Dans la formule de ces monomères, le groupe insaturé est de préférence fixé en position para de l'hydroxyle de la fonction phénol. Leur préparation implique donc par exemple, la fixation du groupe insaturé sur l'acide parahydroxybenzoïque et l'estérification de la fonction phénol par réaction avec le composé à fonction acide constituant le produit actif, dans un ordre qui est en général indifférent.

Dans des formes de mise en oeuvre préférées de l'invention, les monomères utilisés s'obtiennent par réaction de l'indométhacine avec le méthacryloxy-4 phénol, ou de préférence avec le méthacrylamido-4 phénol, dans des conditions propres à former par estérification l'indométhacinate correspondant. La préparation des compositions de l'invention s'effectue ensuite, à partir de ces monomères et des autres constituants, par les techniques de mélange usuelles en la matière.

Selon une caractéristique secondaire de l'invention, il est apparu bénéfique d'incorporer dans la composition un monomère supplémentaire, copolymérisable avec les constituants de l'hydrogel et capable, dans le polymère résultant (qui est alors un terpolymère), d'assurer une assistance anchimérique qui augmente la vitesse d'hydrolyse du produit actif. Dans ce but, on peut avantageusement utiliser un composé insaturé à fonction acide, tel que l'acide acrylique, l'acide méthacrylique, les esters ou amides acryliques ou méthacryliques d'hydroxyoxyalkyle où la chaîne alkylique comporte de 2 à 5 atomes de carbone.

On décrira maintenant l'invention plus en détails dans le cadre d'exemples particuliers de mise en oeuvre qui ne sont nullement limitatifs.

## EXEMPLE I

Dans un premier stade on prépare un monomère estérifié par le composé actif sur une fonction phénol.

Le monomère de départ doit contenir une fonction phénol et un groupe insaturé tel qu'un groupe alkyle insaturé du type vinyle ou allyle ou un groupe ester ou amide acrylique ou méthacrylique. Ce groupe insaturé est de préférence fixé en position para de l'hydroxyle du phénol. Dans le cas particulier considéré ici, on a choisi le méthacrylamido-4 phénol. Par ailleurs, le composé actif est l'indométhacine, médicament connu pour ses propriétés anti-inflammatoires.

A une solution de 0,01 mole (3,58 g) d'indométhacine dans 100 ml de chlorure de méthylène et de 0,011 mole (1,95 g) de méthacrylamido-4 phénol, on ajoute 0,001 mole (0,122 g) de diméthylaminopyridine (DMAP) et 0,011 mole (2,27 g) de dicyclohexylcarbodiimide (DCC). On agite le mélange à la température ambiante, puis pendant 3 h à 30°C.

On filtre le précipité d'urée formé. On lave le filtrat à l'eau (50 ml), puis trois fois avec chaque fois 50 ml de $CH_3COOH$ à 5%, puis enfin à l'eau. On sèche le produit sur $Na_2SO_4$. Après évaporation, on dissout le produit obtenu dans l'acétone, puis on le précipite dans le pentane.

On obtient ainsi 4,19 g (rendement de 81%) d'indométhacinate de méthacrylamido-4 phénol, fondant à 158°C et répondant à l'analyse :

Calculé : C : 67,34 ; H : 4,84 ; Cl : 6,87 ; N : 5,42

Trouvé : C : 67,07 ; H : 4,92 ; Cl : 6,50 ; N : 5,49.

Dans un deuxième stade, ce produit est copolymérisé avec les constituants classiques d'une composition de polymère hydrophile (hydrogel).

A cette fin, ce monomère est ajouté en proportion de 5% par rapport au poids total, à un mélange contenant 29,4 parties en poids de méthacrylate de méthyle et 70,6 parties en poids de N-vinylpyrrolidone.

Après addition de l'amorceur (0,031 g de AIBN ou azo-bis-isobutyronitrile) et de réticulants (0,043% de triéthylène glycol diméthacrylate et 0,157% de triallyl isocyanurate), le mélange est coulé dans des moules, puis la polymérisation est effectuée selon un cycle thermique allant de 40°C à 120°C.

On obtient des rondelles de 0,1 à 0,2 mm d'épaisseur, transparentes et légèrement jaunes.

Ces rondelles sont placées dans 10 ml de tampon phosphate à pH 7 à 37°C (± 0,5°C). Elles sont changées de solution tous les jours et l'on mesure la densité optique de la solution à 319 nm à l'aide d'un spectrophotomètre UV/visible.

L'hydrolyse est lente et se poursuit pendant plus de 44 jours.

La constante d'hydrolyse est de $4{,}610^{-4}$ h$^{-1}$, ce qui conduit dans cet exemple à une dose quotidienne de 10,9 µg d'indométhacine.

## EXEMPLE II

Le monomère indométhacinate préparé dans l'exemple précédent est ajouté en proportion de 5% par rapport au poids total de la composition à un mélange de 29,4 parties en poids de vinylpyrrolidone et 70,6 parties en poids d'hydroxyéthyl-méthacrylate.

Après addition d'amorceur et de réticulants, le mélange est coulé dans des moules, puis la polymérisation est effectuée selon un cycle thermique allant de 40 à 120°C. On obtient des rondelles de 0,1 à 0,2 mm d'épaisseur, transparentes et légèrement jaunes.

Ces rondelles sont placées dans 10 ml de tampon phosphate à pH 7 à 37°C ($\pm$ 0,5°C). Elles sont changées de solution quotidiennement et l'on mesure la densité optique de la solution à 319 nm à l'aide d'un spectrophotomètre UV/visible.

La constante d'hydrolyse mesurée est trouvée égale à $3{,}1.10^{-4}$ h$^{-1}$, ce qui conduit à une dose quotidienne de 8,7 µg d'indométhacine.

Les conditions utilisées sont sensiblement celles que l'on retrouverait dans l'utilisation des rondelles comme lentilles de contact.

D'une manière plus générale, la dose quotidienne d'indométhacine administrée peut être adaptée en fonction des besoins thérapeutiques en faisant varier la proportion du monomère porteur du médicament entre 1 et 10% en poids par rapport au poids total de la composition.

## EXEMPLE III

On procède comme dans l'exemple précédent, sauf que l'on ajoute à la composition de l'acide acrylique ou de l'acide méthacrylique, dans une proportion variant de 1 à 10% en poids, par rapport au poids total de la composition.

Ceci permet d'augmenter la vitesse d'hydrolyse libérant l'indométhacine. Par exemple, si l'on ajoute 1% d'acide méthacrylique, la vitesse d'hydrolyse est de $7{,}2.10^{-4}$ h$^{-1}$.

Une proportion au plus égale à 5% en poids de comonomère acide, par rapport au poids total des divers monomères dans la composition globale, semble particulièrement appropriée pour les lentilles de contact ainsi fabriquées.

## EXEMPLE IV

En reprenant la même procédure que dans les exemples précédents, on remplace le méthacrylamido-4 phénol de l'étape de l'Exemple I par le méthacryloxy-4 phénol. Par ailleurs le monomère indométhacinate est utilisé dans les proportions de 4% et 6% en poids dans le poids total du mélange à polymériser. On obtient sensiblement les mêmes résultats.

## EXEMPLE V

La procédure des exemples I et II et IV est appliquée en remplaçant l'indométhacine, répondant à la formule de l'acide 1-(p-chlorobenzoyl)-5-méthoxy-2-méthyl-indole-3-acétique, par :
— le fentiazac, ou acide 2 aza-1-phényl-3(p-chlorophényl)thiényl-5-acétique ;
— le suprofène, ou acide alpha-méthyl-4(2-thiénylcarbonyl-benzène-acétique.

L'un comme l'autre conduisent à des polymères à effet anti-inflammatoire.

On remarque que comme la molécule de l'indométhacine, celle de ces deux composés actifs comportent un groupement acide sur un radical alkyle à 2 à 5 atomes de carbone et un groupement arylique comportant au moins un cycle phényle et au moins un hétérocycle où l'hétéro-atome est N ou S.

Naturellement, l'invention n'est en rien limitée par les particularités qui ont été spécifiées dans les exemples qui précèdent ou par les détails des modes de mise en oeuvre particuliers choisis pour illustrer l'invention.

## Revendications

1. Composition de polymère support de produit actif relargable caractérisée en ce qu'elle comporte des constituants classiques d'un hydrogel et un monomère porteur de produit actif copolymérisable avec lesdits constituants, ledit monomère comportant un radical aryle dont une fonction phénol est estérifiée par un composé à fonction acide constituant ledit produit actif.

2. Composition selon la revendication 1, caractérisée en ce que ledit composé est du type de l'indométhacine et notamment choisi parmi l'indométhacine, le fentaziac et le suprofène.

3. Composition selon la revendication 1 ou 2, caractérisée en ce que ledit monomère comporte un groupe insaturé en position para de l'hydroxyle de la fonction phénol.

4. Composition selon la revendication 1, 2 ou 3, caractérisée en ce que ledit monomère est un ester ou amide acrylique ou méthacrylique.

5. Composition selon la revendication 4, caractérisée en ce que ledit monomère est l'indométhacinate de méthacrylamido-4 phénol.

6. Composition selon l'une quelconque des revendications 1 à 5, caractérisée en ce que lesdits constituants sont ceux d'un hydrogel du type convenant à la fabrication de lentilles de contact, comme le méthacrylate de méthyle, le méthacrylate d'hydroxyéthyle (HEMA), le méthacrylate de glycéryle, la N-vinylpyrrolidone, l'acide acrylique et l'acide méthacrylique, ces différents composés étant utilisés soit seuls, soit en mélange, et sous forme de monomères, d'homopolymères ou de copolymères.

7. Composition selon l'une quelconque des revendications 1 à 6, caractérisée en ce que ledit produit actif est présent dans une proportion comprise entre 1 et 10% en poids, notamment entre 2 et 6% en poids, par rapport au poids total de la composition.

8. Composition selon l'une quelconque des revendications 1 à 7, caractérisée en ce qu'elle comporte en outre un comonomère à fonction acide.

9. Matériaux polymères supports de produits actifs relargables tels qu'obtenus par mise en forme et polymérisation d'une composition selon l'une quelconque des revendications 1 à 8.

10. Matériaux selon la revendication 9, caractérisés en ce qu'ils sont sous forme de lentilles de contact.

11. Procédé de préparation de matériaux suivant la revendication 9 ou 10, caractérisé en ce qu'il comporte la préparation dudit monomère porteur de produit actif, notamment par réaction de l'indométhacine avec le méthacrylamido-4 phénol, dans des conditions propres à former l'ester correspondant, le mélange dudit monomère avec les autres constituants de ladite composition, et la polymérisation de la composition obtenue après addition d'un amorceur de polymérisation et d'un réticulant de manière en soi connue.

**Patentansprüche**

1. Polymerträger-Zusammensetzung eines aktiven abspaltbaren Produkts, dadurch gekennzeichnet, daß sie die klassischen Bestandteile eines Hydrogels und einen monomeren Träger des aktiven Produkts enthält, der mit diesen Bestandteilen copolymerisierbar ist, wobei das genannte Monomere eine Arylgruppe trägt, bei welcher eine Phenolfunktion mit einer eine saure Funktion tragenden Verbindung verestert ist, die dieses aktive Produkt darstellt.

2. Zusammensetzung nach Anspruch 1, dadurch gekennzeichnet, daß die Verbindung vom Indometacin-Typ ist und insbesondere unter Indometacin, Fentaziac und Suprofen ausgewählt ist.

3. Zusammensetzung nach Anspruch 1 oder 2, dadurch gekennzeichnet, daß das Monomere in para-Stellung zur Hydroxy-Gruppe der Phenolfunktion eine ungesättigte Gruppe trägt.

4. Zusammensetzung nach Anspruch 1, 2 oder 3, dadurch gekennzeichnet, daß das Monomere ein Acrylsäure- oder Methacrylsäureester oder -amid ist.

5. Zusammensetzung nach Anspruch 4, dadurch gekennzeichnet, daß das Monomere der Indometacinester von Methacrylamido-4-phenol ist.

6. Zusammensetzung nach einem der Ansprüche 1 bis 5, dadurch gekennzeichnet, daß die Bestandteile diejenigen eines Hydrogels von der Art sind, das sich für die Herstellung von Kontaktlinsen eignet, zum Beispiel Methacrylsäuremethylester, Methacrylsäurehydroxyethylester (HEMA), Methacrylsäureglycerid, N-Vinylpyrrolidon, Acrylsäure und Methacrylsäure, wobei diese verschiedenen Verbindungen entweder allein oder in Mischung eingesetzt werden, und zwar in Form von Monomeren, Homopolymeren oder Copolymeren.

7. Zusammensetzung nach einem der Ansprüche 1 bis 6, dadurch gekennzeichnet, daß das aktive Produkt in einem Anteil vorliegt, der 1 bis 10 Gew.-%, vorzugsweise 2 bis 6 Gew.-%, bezogen auf das Gesamtgewicht der Zusammensetzung, umfaßt.

8. Zusammensetzung nach einem der Ansprüche 1 bis 7, dadurch gekennzeichnet, daß sie außerdem ein Comonomeres mit einer sauren Funktion enthält.

9. Polymerträger-Materialien aktiver abspaltbarer Produkte, erhältlich durch In-Form-Bringen und Polymerisation einer Zusammensetzung nach einem der Ansprüche 1 bis 8.

10. Materialien nach Anspruch 9, dadurch gekennzeichnet, daß sie in Form von Kontaktlinsen vorliegen.

11. Verfahren zum Herstellen von Materialien nach Anspruch 9 oder 10, dadurch gekennzeichnet, daß es die Herstellung des genannten monomeren Trägers des aktiven Produkts, insbesondere durch Umsetzung von Indometacin mit Methacrylamido-4-phenol unter den Bedingungen, die sich für die Bildung des entsprechenden Esters eignen, die Mischung des genannten Monomeren mit den anderen Bestandteilen der Zusammensetzung und die Polymerisation der Zusammensetzung, die nach dem Zusatz eines Polymerisationsinitiators und eines Vernetzers in an sich bekannter Weise erhalten wurde, umfaßt.

**Claims**

1. A composition of a polymer support for a releasable active material characterised in that it comprises the conventional constituents of a hydrogel and a monomer bearing the active material which can be copolymerised with the said constituents, the said monomer incorporating an aryl radical whose phenol group is esterified by a compound having an acid group constituting the said active material.

2. A composition according to claim 1, characterised in that the said compound is of the indomethacin type, and is in particular selected from indomethacin, fentaziac and suprofen.

3. A composition according to claim 1 or 2, characterised in that the said monomer comprises an unsaturated group in the para position to the hydroxyl group of the phenol.

4. A composition according to claim 1, 2 or 3, characterised in that the said monomer is an acrylic or methacrylic ester or amide.

5. A composition according to claim 4, characterised in that the said monomer is methacrylamido-4-phenol indomethacinate.

6. A composition according to any one of claims 1 to 5, characterised in that the said components are those of a hydrogel of a type suitable for the manufacture of contact lenses, such as methyl methacrylate, hydroxethyl methacrylate (HEMA), glyceryl methacrylate, N-vinylpyrrolidone, acrylic acid and methacrylic acid, these various components being used either alone or as a mixture and in the form of monomers, homopolymers and copolymers.

7. A composition according to any one of claims 1 to 6, characterised in that the said active material is present in a proportion of between 1 and 10% by weight, in particular between 2 and 6% by weight, with respect to the total weight of the composition.

8. A composition according to any one of claims 1 to 7, characterised in that it also comprises a comonomer having an acid group.

9. Polymer material supports for releasable active materials such as are obtained by the contitution and polymerisation of a composition according to any one of claims 1 to 8.

10. Materials according to claim 9, characterised in that these are in the form of contact lenses.

11. A method for preparing materials according to claims 9 or 10, characterised in that it comprises preparation of the said monomer bearing the active material, in particular by the reaction between indomethacin and methacrylamido-4-phenol under conditions such as to form the corresponding ester, mixing the said monomer with the other constituents of the said composition and polymerisation of the composition obtained after the addition of a polymerisation initiator and a cross-linking agent in a manner which is in itself known.